# EUROPEAN PATENT SPECIFICATION

(11) **EP 3 980 111 B1**
(45) Date of publication and mention of the grant of the patent: **18.03.2026**
(21) Application number: 20716579.6
(22) Date of filing: 18.03.2020
(51) Int. Cl.: A61M 37/00

(54) **MICROARRAY CARRIER FOR MICROARRAY APPLICATOR**
MIKROARRAY-TRÄGER FÜR MIKROARRAY-APPLIKATOR
SUPPORT DE MICRORÉSEAU DESTINÉ À UN APPLICATEUR DE MICRORÉSEAU

(30) Priority: 06.06.2019 US 201962857988 P
(43) Date of publication of application: 13.04.2022
(73) Proprietor: Kindeva Drug Delivery L.P., Woodbury, MN 55129 (US)
(72) Inventor: LEY, Gregory R., Saint Paul, Minnesota 55133-3427 (US); SELBY, Robert G.M., Melbourn Royston Hertfordshire SG8 6DT (GB); SAVOV, Svilen S., Cambridge Cambridgeshire CB4 1DT (GB)
(74) Representative: Vossius & Partner Patentanwälte Rechtsanwälte mbB
(86) International application number: PCT/IB2020/052478
(87) International publication number: WO 2020/245667

(56) References cited:
- WO-A1-2006/055771
- WO-A1-2019/040063
- WO-A1-2019/077519

## Description

### Technical Field

The present disclosure relates generally to a microarray carrier for use with a microarray applicator.

### Background

Transdermal and topical drug delivery can be used for therapeutic treatment, but the number of molecules that can be effectively delivered using these routes can be limited by the barrier properties of skin. The main barrier to transport of molecules through the skin is the stratum corneum (the outermost layer of the skin). A number of different skin treatment methods have been proposed in order to increase the permeability or porosity of the outermost skin layers, such as the stratum corneum, thus enhancing drug delivery through or into those layers. Devices including arrays of relatively small structures, sometimes referred to as microneedles or micro-pins, have been disclosed for use in connection with the delivery of therapeutic agents and other substances through the skin and other surfaces. The devices can be pressed against the skin in an effort to pierce the stratum corneum, such that the therapeutic agents and other substances can sequentially or simultaneously pass through that layer and into the tissues below. Microneedles of these devices pierce the stratum corneum upon contact, making a plurality of microscopic openings which serve as passageways through which molecules of active components can be delivered into the body.

Microneedle arrays and patches can be deployed with an applicator capable of being used a number of different times. The microneedle arrays and patches are generally used once and then discarded. The applicator devices can be repeatedly reloaded with new microneedle arrays and patches. Microneedle array applicators may be used to deposit a microarray patch on a substrate, such as a skin surface, during a transdermal drug delivery procedure. The microarray applicator includes a microarray carrier to support the microarray patch within the microarray applicator. The microarray patch may be supported on a plurality of support surfaces provided on the microarray carrier. In use the microarray applicator pushes the patch from the microarray carrier and onto the skin, for instance, with a spring-driven piston.

WO 2019/077519 A1 discloses an applicator for applying a microneedle array to skin. The applicator can include a body having a first portion and a second portion defining a cavity, the second portion having a slot presented on an outside surface for insertion of the microneedle array into the cavity. The applicator includes a slot or opening for insertion of a microneedle array carrier. The microarray carrier comprises a plurality of tabs disposed on the body and extending into the cavity.

WO 2006/055771 A1 discloses an applicator used to apply microneedle arrays. An applicator capable of sensing a controlled distance from a skin surface and propelling a microneedle array across this distance and into the skin surface is disclosed. Another applicator used to apply microneedle arrays is disclosed in WO 2019/040063 A1.

### Summary

In some cases, the microarray patch may adhere unevenly to the support surfaces of the microarray carrier during detachment of the microarray patch from the microarray carrier. This is particularly true when the microarray patch is not placed precisely in the center of the microarray carrier. As such, the microarray patch may be released unevenly or may be off-centered with respect to the microarray carrier, resulting in an uneven application of the microarray patch and uneven drug delivery to the substrate. Hence, there is a need for an improved microarray carrier for such applications, and particularly a carrier that provides for ejection of the patch in a more repeatable way, with less variation in the placement of the patch on the subject from application to application.

In one aspect, a microarray carrier for use with a microarray applicator is provided. The microarray carrier includes a body defining a cavity. The microarray carrier also includes a plurality of tabs disposed on the body and extending into the cavity. Each of the plurality of tabs defines a contour line extending along a length thereof from a proximal end of the tab adjacent the body to a support surface spaced apart from the body. The support surface is inclined with respect to the contour line where the contour line intersects the support surface. A projected area of each of the plurality of tabs onto a horizontal plane of the cavity is greater than an area of the support surface. Each support surface is aligned non-perpendicular to the horizontal plane. As such, each of the tabs provides a defined contact area and adhesion between each of the plurality of tabs and a microarray patch, in turn, providing a centered and even release of the microarray patch from each of the plurality of tabs. This further provides uniform application of the microarray patch and uniform drug delivery onto a skin surface of a subject.

In another aspect, a microarray applicator is provided. The microarray applicator includes a microarray carrier. The microarray applicator also includes a housing. The microarray applicator further includes a plunger adapted to be movably disposed within the housing. The microarray carrier is removably and at least partially disposed within the housing. The microarray applicator may be reused with multiple and/or new microarray patches, in turn, improving usability and reducing cost.

A method of using a microarray applicator is disclosed. The method includes releasably receiving a microarray patch on a microarray carrier. The method also includes removably receiving the microarray carrier with the microarray patch at least partially within a housing. The method further includes moving the plunger from a first position to a second position in order to release the microarray patch from the microarray carrier and eject the microarray patch from the housing.

The invention is defined by claim 1 and further defined by the dependent claims.

### Brief Description of the Drawings

Exemplary embodiments disclosed herein may be more completely understood in consideration of the following detailed description in connection with the following figures. The figures are not necessarily drawn to scale. Like numbers used in the figures refer to like components. However, it will be understood that the use of a number to refer to a component in a given figure is not intended to limit the component in another figure labeled with the same number.
FIG. 1 is a partial cutaway perspective view of a microarray applicator, according to one embodiment of the present disclosure;
FIG. 2A is a perspective view of a microarray carrier of the microarray applicator of FIG. 1 without a microarray patch;
FIG. 2B is a bottom perspective view of the microarray carrier of FIG. 2A with the microarray patch;
FIGS. 3A and 3B are side and perspective views, respectively, of a tab of the microarray carrier of FIG. 2;
FIGS. 3C to 3i are side views of different embodiments of the tab of the microarray carrier of FIG. 2;
FIG. 4 is a bottom perspective view of another microarray carrier of the microarray applicator of FIG. 1 with the microarray patch;
FIGS. 5A and 5B are partial cutaway perspective views of the microarray applicator of FIG. 1 showing different operating positions; and
FIG. 6 is a flowchart of a method of using the microarray applicator of FIG. 1.

### Detailed Description

In the following description, reference is made to the accompanying figures that form a part thereof and in which various embodiments are shown by way of illustration. It is to be understood that other embodiments are contemplated and may be made without departing from the scope of the present disclosure. The following detailed description, therefore, is not to be taken in a limiting sense.

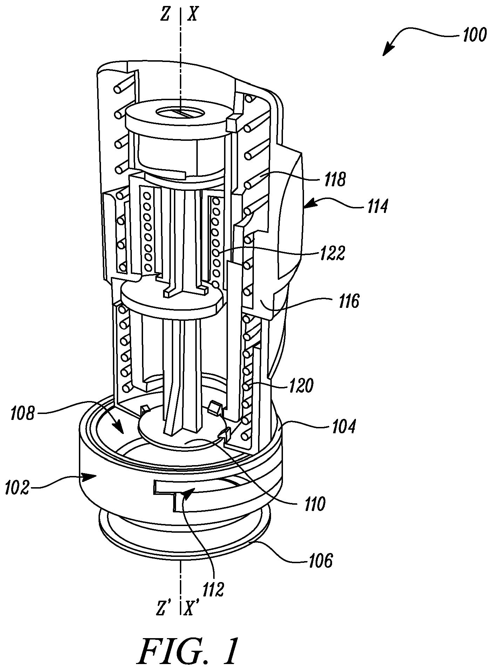

The present disclosure relates to a microarray carrier that can be used with a microarray applicator. The microarray applicator is, in turn, primarily used for medical applications. For example, the microarray applicator may be used in conjunction with the microarray carrier to deposit a microarray patch on a skin surface of a subject during a transdermal drug delivery procedure. Referring to FIG. 1, a partial cross-sectional view of a microarray applicator 100 is illustrated. The microarray applicator 100 includes a housing 102. In the illustrated embodiment, the housing 102 has a substantially hollow and circular configuration defining an axis X-X'. In other embodiments, the housing 102 may have any other configuration, such as triangular, rectangular, elliptical, and so on, based on application requirements. The housing 102 includes a first major surface 104 and a second major surface 106. The second major surface 106 is disposed axially opposite to the first major surface 104 relative to the axis X-X'.

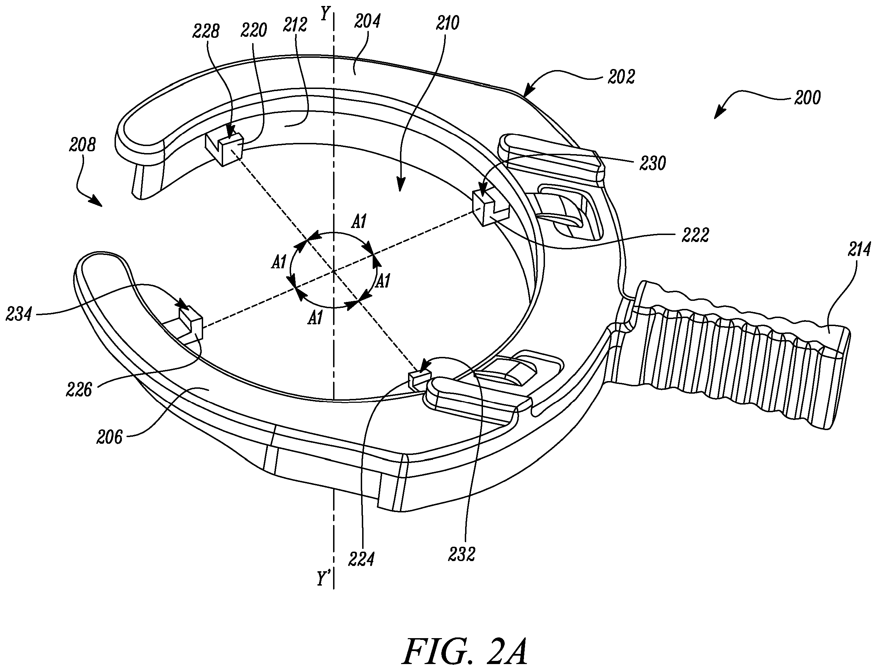

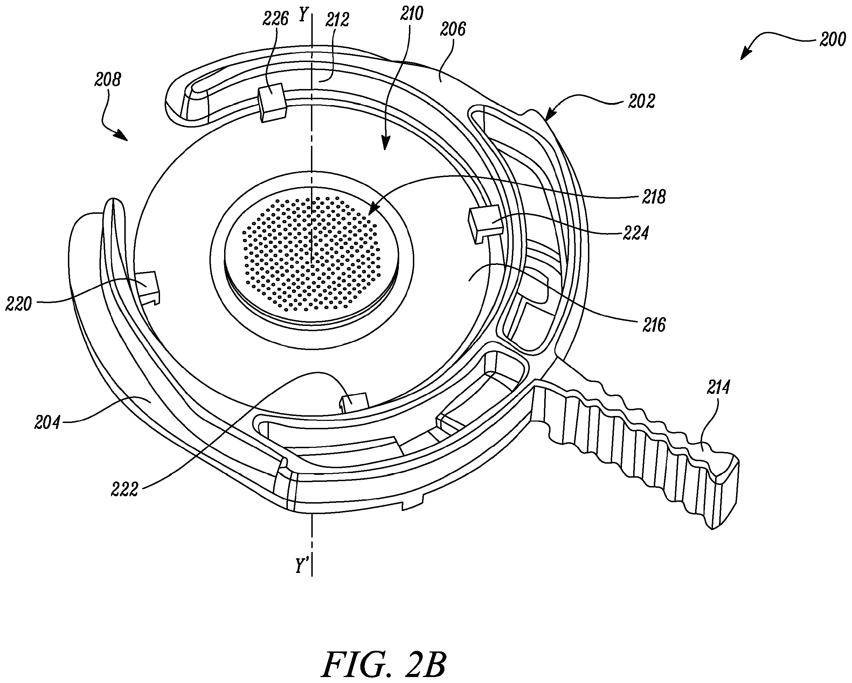

The housing 102 also includes a bore 108. The bore 108 extends centrally along the axis X-X' from the first major surface 104 toward the second major surface 106. In the illustrated embodiment, the bore 108 has a substantially circular configuration. In other embodiments, the bore 108 may have any other configuration, such as triangular, rectangular, elliptical, and so on, based on application requirements. The bore 108 is adapted to movably receive a plunger 110. The housing 102 also includes a receiving portion 112. The receiving portion 112 has a substantially hollow configuration. The receiving portion 112 is disposed in communication with the bore 108 and, more specifically, between the first major surface 104 and the second major surface 106. The receiving portion 112 is adapted to removably receive a microarray carrier 200 (the microarray carrier 200 is not shown in FIG.1, but it is visible in FIG. 2).

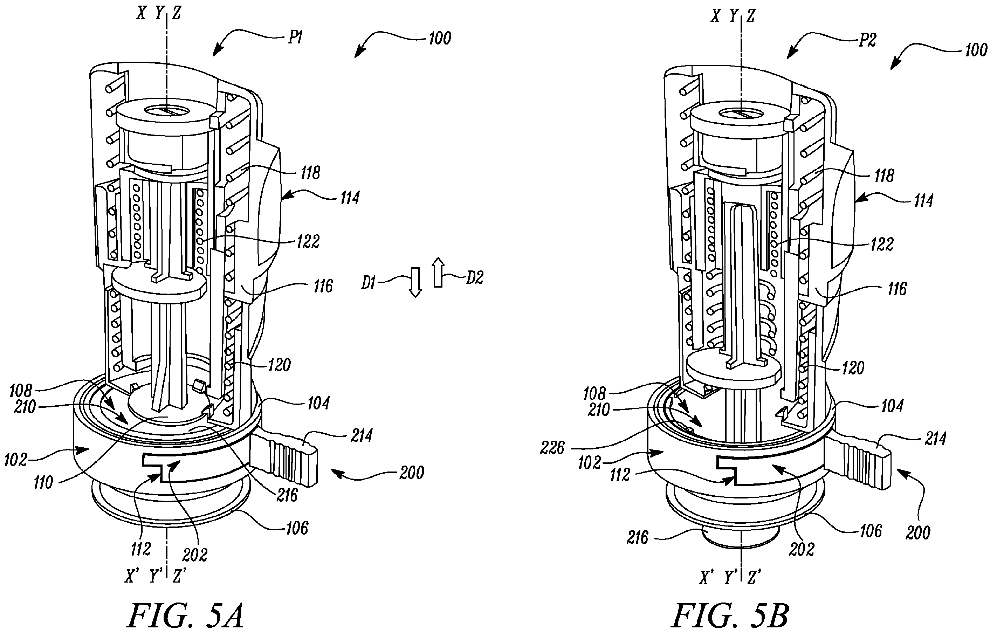

The microarray applicator 100 also includes a plunger system 114 disposed on the housing 102. In Figures 1, 5A and 5B, a cross-sectional view of the plunger system 114 is illustrated. The plunger system 114 includes an enclosure 116. In the illustrated embodiment, the enclosure 116 has a substantially cylindrical and hollow configuration. In other embodiments, the enclosure 116 may have any other configuration, such as triangular, rectangular, elliptical, and so on, based on application requirements. The enclosure 116 is adapted to be disposed on the first major surface 104 of the housing 102. The plunger system 114 also includes the plunger 110 and a number of springs 118, 120, 122 disposed within the enclosure 116. As such, the plunger 110 is adapted to be movably disposed within the enclosure 116 and further within the bore 108 of the housing 102. Accordingly, in the illustrated embodiment, the plunger 110 has a substantially circular configuration defining an axis Z-Z'. In other embodiments, the plunger 110 may have any other configuration, such as triangular, rectangular, elliptical, and so on, based on the configuration of the bore 108.

Referring to FIG. 2A, a perspective view of one embodiment of the microarray carrier 200 is illustrated. Referring to FIG. 2B, a bottom perspective view of the microarray carrier 200 is illustrated. The microarray carrier 200 will now be explained with combined reference to FIGS. 2A and 2B. The microarray carrier 200 includes a body 202 defining an axis Y-Y'. The body 202 is adapted to be removably disposed within the housing 102. More specifically, the body 202 of the microarray carrier 200 is adapted to be removably received in the receiving portion 112 of the housing 102, such that the axis Y-Y' coincides with the axis X-X'. Accordingly, in the illustrated embodiment, the body 202 has a substantially annular configuration. In other embodiments, the body 202 may have any other configuration, such as triangular, rectangular, elliptical, and so on, based on the configuration of the housing 102. The body 202 includes a first arm 204 and a second arm 206. The second arm 206 is coplanar relative to the first arm 204. Each of the first arm 204 and the second arm 206 has a substantially curved configuration. Also, a gap 208 is defined in the body 202 between the first arm 204 and the second arm 206. The gap 208 is adapted to provide a clearance for movement of the first arm 204 and the second arm 206 toward one another during inserting and removing the microarray carrier 200 from the housing 102.

The body 202 also defines a cavity 210 disposed between the first arm 204 and the second arm 206. The cavity 210 is provided in communication with the gap 208. The cavity 210 is defined by an inner surface 212 of the body 202. In the illustrated embodiment, the cavity 210 has a substantially annular configuration. In other embodiments, the cavity 210 may have any other configuration, such as triangular, rectangular, elliptical, and so on, based on the configuration of the first arm 204 and the second arm 206. The microarray carrier 200 also includes a handle 214 extending from the body 202. In the illustrated embodiment, the handle 214 has a substantially elongate configuration. In other embodiments, the handle 214 may have any other configuration, such as curved, rectangular, and so on, based on application requirements. The handle 214 may allow the microarray carrier 200 to be manipulated, for example, for inserting and removing the microarray carrier 200 from the housing 102.

The microarray carrier 200 also includes a microarray patch 216. The microarray patch 216 is disposed releasably within the cavity 210. In the illustrated embodiment, the microarray patch 216 has a substantially circular configuration. In other embodiments, the microarray patch 216 may have any other configuration, such as triangular, rectangular, elliptical, and so on, based on the configuration of the cavity 210. The microarray patch 216 includes one or more microneedles 218 extending away from the microarray patch 216. In the illustrated embodiment, the microneedles 218 are disposed in a substantially octagonal configuration on the microarray patch 216. **In** other embodiments, the microneedles 218 may be disposed in any other configuration on the microarray patch 216, such as circular, triangular, rectangular, elliptical, and so on, based on application requirements. The microneedles 218 may have any configuration, such as solid, hollow, or a combination of solid and hollow, and so on.

It should be noted that the microarray patch 216 and the microneedles 218 described herein are merely exemplary and may vary, based on application requirements. For example, in some embodiments, the microarray patch 216 may include one or more microprotrusions. In some embodiments, the microneedles 218 or the microprotusions may be dissolvable type microneedles or microprotusions, respectively, adapted to dissolve within the skin surface of the subject after application. In some embodiments, the microneedles 218 or the microprotusions may be coated or filled with one or more types of drug formulations to be administered through the skin surface of the subject. In yet some embodiments, the microarray patch 216 may be a transdermal microarray patch adapted to be deposited on the skin surface of the subject, such that the microneedles 218 or the microprotusions may be omitted.

The microarray carrier 200 further includes a plurality of tabs 220, 222, 224, 226 disposed on the inner surface 212 of the body 202 and extending into the cavity 210. The plurality of tabs 220, 222, 224, 226 is adapted to releasably support the microarray patch 216 within the cavity 210. More specifically, each of the tabs 220, 222, 224, 226 includes a support surface 228, 230, 232, 234, respectively, spaced apart from the body 202. Accordingly, the support surface 228, 230, 232, 234 of each of the plurality of tabs 220, 222, 224, 226 is adapted to releasably support the microarray patch 216 thereon. In the illustrated embodiment, the plurality of tabs 220, 222, 224, 226 includes four tabs 220, 222, 224, 226. Each of the tabs 220, 222, 224, 226 is disposed on the inner surface 212 of the body 202. Further, the tabs 220, 222, 224, 226 are disposed equiangularly around the cavity 210. As such, an angle "A1" between the adjoining tabs 220, 222, 224, 226 measures approximately 90 degrees (°). In other embodiments, an actual value of the angle "A1" between the adjoining tabs 220, 222, 224, 226 may vary, based on application requirements.

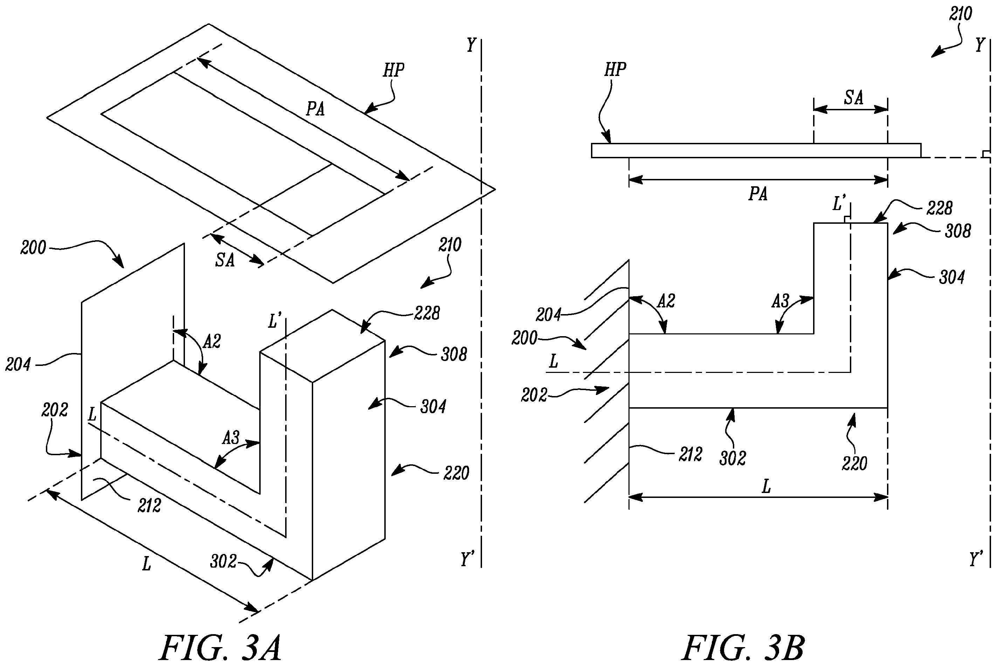

Referring to FIG. 3A, an exemplary perspective view of a portion of the cavity 210 and the tab 220 disposed therein is illustrated. The accompanying figure also illustrates an exemplary horizontal plane "HP" of the cavity 210. The horizontal plane "HP" is disposed within the cavity 210, such that the horizontal plane "HP" is substantially normal to the axis Y-Y'. As shown in the accompanying figure, a projected area "PA" of the tab 220 on the horizontal plane "HP" of the cavity 210 is greater than an area "SA" of the support surface 228. The projected area "PA" is substantially equal to an area of a top view of the tab 220. In a conventional microarray carrier (not shown) having one or more conventional tabs (not shown), each of the conventional tabs provides a support surface for the microarray patch 216 substantially equal to the projected area "PA". More specifically, in the conventional carrier, the microarray patch 216 may rest on a substantial portion of the conventional tab, in turn, increasing a contact area and adhesion between the microarray patch 216 and each of the conventional tabs. If the patch is asymmetrically aligned in the support cavity, then the contact area between patch and each individual tab may vary. The support surface 228 is also substantially parallel to the horizontal plane "HP" of the cavity 210. Further, the axis Y-Y' is substantially normal to the support surface 228.

As shown in FIG. 3A, the tab 220 provides a contact area with the microarray patch 216 substantially equal to the area "SA" of the support surface 228, such that the area "SA" of the support surface 228 is merely a fraction of the projected area "PA" of the tab 220. Accordingly, the support surface 228 provides a predetermined contact area and friction between the microarray patch 216 and each tab 220, in turn, providing substantially improved, even, and centered detachment of the microarray patch 216 from the microarray carrier 200. As such, a possibility of undesired sticking of the microarray patch 216 to one or more tabs 220 and/or the microarray carrier 200 is substantially reduced. This results in a substantially improved and uniform application of the microarray patch 216 on the skin surface of the subject, in turn, providing improved and uniform drug delivery via the microneedles 218.

Referring to FIG. 3B, a schematic side view of the tab 220 is illustrated. The tab 220 defines a contour line L-L' extending along a length "L" thereof. The contour line L-L' is substantially normal to a cross-sectional surface of the tab 220 along the length "L". Depending on a shape of the tab 220, the contour line L-L' may be linear, curved, substantially L-shaped, and so forth. The tab 220 includes a first portion 302 and a second portion 304. The first portion 302 extends from the inner surface 212 of the body 202 into the cavity 210. The first portion 302 defines an angle "A2" relative to the inner surface 212. In the illustrated embodiment, the angle "A2" measures approximately 90°. In other embodiments, an actual value of the angle "A2" may vary, based on application requirements. The second portion 304 extends from the first portion 302 and defines the support surface 228. The second portion 304 is inclined to the first portion 302 and defines an angle "A3" relative to the first portion 302. In the illustrated embodiment, the angle "A3" measures approximately 90°. In other embodiments, an actual value of the angle "A3" may vary, based on application requirements. As such, in some situations, the angle "A3" may range from about 80° to about 100°.

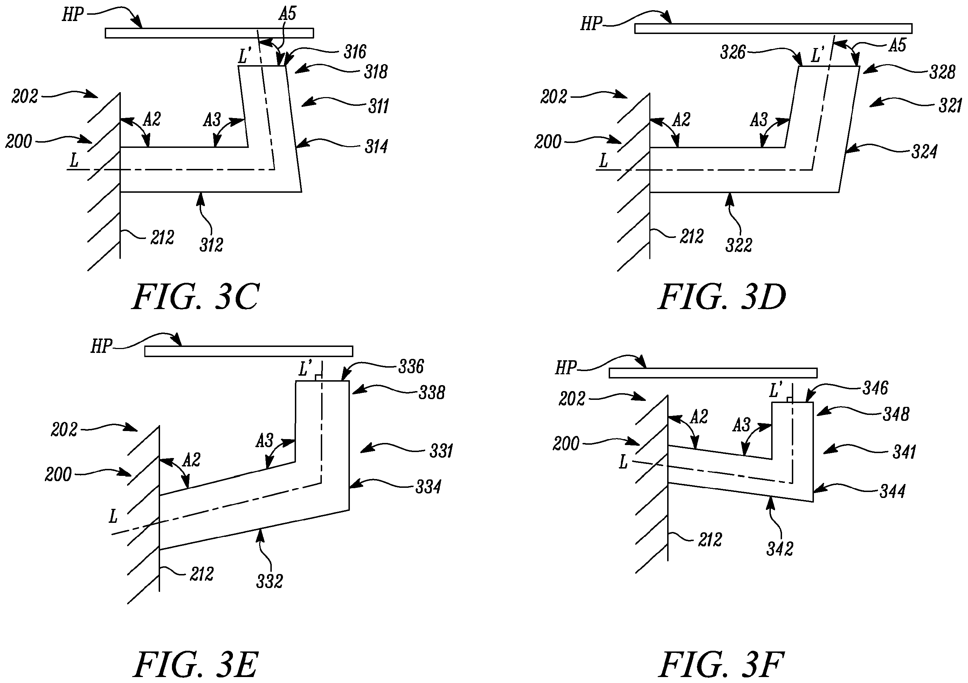

For example, referring to FIG. 3C, a tab 311 includes a first portion 312, a second portion 314, and a support surface 316 provided at an end 318 on the second portion 314. In the illustrated example, the angle "A3" measures approximately 80°. Also, the angle "A2" measures approximately 90°. In another embodiment, referring to FIG. 3D, a tab 321 includes a first portion 322, a second portion 324, and a support surface 326 provided at an end 328 on the second portion 324. In the illustrated example, the angle "A3" measures approximately 100°. Also, the angle "A2" measures approximately 90°. In another embodiment, referring to FIG. 3E, a tab 331 includes a first portion 332, a second portion 334, and a support surface 336 provided at an end 338 on the second portion 334. In the illustrated example, the angle "A3" measures approximately 100°. Also, the angle "A2" measures approximately 80°. In another embodiment, referring to FIG. 3F, a tab 341 includes a first portion 342, a second portion 344, and a support surface 346 provided at an end 348 on the second portion 344. In the illustrated example, the angle "A3" measures approximately 80°. Also, the angle "A2" measures approximately 100°.

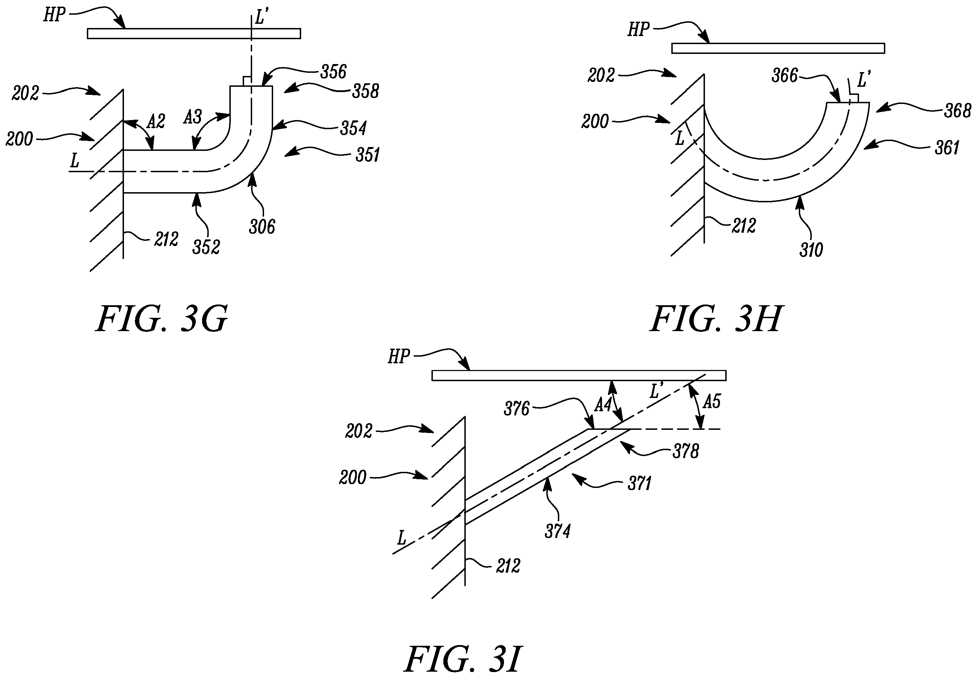

In some embodiments, a tab 351 may be at least partially curved. For example, referring to FIG. 3G, the tab 351 includes a first portion 352 connected to a second portion 354 via a curved portion 306, and a support surface 356 provided at an end 358 on the second portion 354. In another embodiment, referring to FIG. 3H, a tab 361 has a substantially C-shaped configuration, such that a support surface 366 is disposed on an end 368 of the tab 361 distal to the body 202. In such a situation, the first portion and the second portion are combined into a single unitary portion 310. In some embodiments, a tab 371 may be inclined obliquely relative to the horizontal plane "HP" of the cavity 210. The horizontal plane "HP" is substantially normal to the axis Y-Y'. For example, referring to FIG. 3I, the tab 371 includes a second portion 374 directly connected to the inner surface 212, and a support surface 376 provided at an end 378 on the second portion 374. In such a situation, the first portion is omitted. Also, the second portion 374 defines an angle "A4" relative to the horizontal plane "HP". In the illustrated embodiment, the angle "A4" measures approximately 45°. In other embodiments, an actual value of the angle "A4" may vary based on application requirements.

The support surface is aligned non-perpendicularly with respect to the horizontal plane. In some embodiments the support surface is aligned at an angle of less than 45 degrees, less than 30 degrees, or less than 10 degrees. In some embodiments, such as those described herein with reference to FIGS. 3A to 3I, the support surface 228, 316, 326, 336, 346, 356, 366, 376 is substantially parallel to the horizontal plane "HP". Also, in each of the embodiments described herein with reference to FIGS. 3A, 3B, 3E, 3F, 3G, and 3H, the contour line L-L' at the distal end of the tab is substantially normal to the support surface 228, 336, 346, 356, 366. In another embodiment, referring to FIGS. 3C, 3D, and 3I, the contour line L-L' at the distal end of the tab is inclined relative to the support surface 316, 326, 376 defining an angle "A5". In the illustrated example of FIG. 3C, the angle "A5" measures approximately 110°. In the illustrated example of FIG. 3D, the angle "A5" measures approximately 80°. In the illustrated example of FIG. 3I, the angle "A5" measures approximately 45°. In other embodiments, an actual value of the angle "A5" may vary based on application requirements.

In some embodiments, the area of the support surface of each tab may be greater than 0.1, 0.2, greater than 0.3, or even greater than 0.4 mm². In some embodiments, the area of the support surface of each tab may be less than 1.5, less than 1.0, less than 0.8, or even less than 0.6 mm². In some embodiments, the area of the support surface of each tab is 0.2 mm² to 1.0 mm². In some embodiments, the area of the support surface of each tab is 0.4 mm² to 0.6 mm².

Also, in the illustrated embodiments, referring to FIGS. 3A to 3I, the support surface 228, 316, 326, 336, 346, 356, 366, 376 is disposed at the end 308, 318, 328, 338, 348, 358, 368, 378 of the tab 220, 311, 321, 331, 341, 351, 361, 371, respectively. In other embodiments, the support surface 228, 316, 326, 336, 346, 356, 366, 376 may be disposed at any location along the length "L" of the tab 220, 311, 321, 331, 341, 351, 361, 371, respectively. In such a situation, a location of the second portion 304, 314, 324, 334, 344, 354, 374 relative to the first portion 302, 312, 322, 332, 342, 352 may also vary along the length "L" of the tab 220, 311, 321, 331, 341, 351, 361, 371, respectively. It should be noted that the various configurations of the tab 220, 311, 321, 331, 341, 351, 361, 371 described herein are merely exemplary and may vary, based on application requirements. It should also be noted that although various possible configurations are described herein with reference to the tab 220, 311, 321, 331, 341, 351, 361, 371, other tabs 222, 224, 226 may also have any configuration as described with reference to FIGS. 3A to 3I. Also, in some embodiments, each of the tabs 220, 222, 224, 226 may have a different configuration with respect to one another as described with reference to FIGS. 3A to 3I, or any other configuration not described herein. Additionally, the first portion 302, 312, 322, 332, 342, 352 and the second portion 304, 314, 324, 334, 344, 354, 374 provides a substantially hook-shaped configuration of the tab 220, 311, 321, 331, 341, 351, 361, 371, thus, providing for uniform adhesion between the microarray patch 216 and the support surface 228, 230, 232, 234, 316, 326, 336, 346, 356, 366, 376 of each of the tabs 220, 222, 224, 226, 311, 321, 331, 341, 351, 361, 371, respectively.

In the illustrated embodiment, each of the tabs 220, 222, 224, 226, 311, 321, 331, 341, 351, 361, 371 have a substantially rectangular cross-sectional configuration. In other embodiments, one or more of the tabs 220, 222, 224, 226, 311, 321, 331, 341, 351, 361, 371 may have any other cross-sectional configuration, such as triangular, circular, elliptical, and so on, based on application requirements. Also, in the illustrated embodiment, the support surface 228, 230, 232, 234, 316, 326, 336, 346, 356, 366, 376 of each of the tabs 220, 222, 224, 226, 311, 321, 331, 341, 351, 361, 371 has a substantially planar configuration. In other embodiments, the support surface 228, 230, 232, 234, 316, 326, 336, 346, 356, 366, 376 of one or more of the tabs 220, 222, 224, 226, 311, 321, 331, 341, 351, 361, 371 may have any other configuration, such as curved, inclined, and so on, based on application requirements. In some embodiments, the support surface 228, 230, 232, 234, 316, 326, 336, 346, 356, 366, 376 of one or more of the tabs 220, 222, 224, 226, 311, 321, 331, 341, 351, 361, 371 may have one or more additional surface elements (not shown), such as one or more protrusions, depressions, ridges, grooves, serrations, and so on, based on application requirements. In yet other embodiments, the support surface 228, 230, 232, 234, 316, 326, 336, 346, 356, 366, 376 of one or more of the tabs 220, 222, 224, 226, 311, 321, 331, 341, 351, 361, 371 may have one or more additional surface textures or finishes (not shown), such as rough, smooth, matt, wavy, knurled, and so on, based on application requirements.

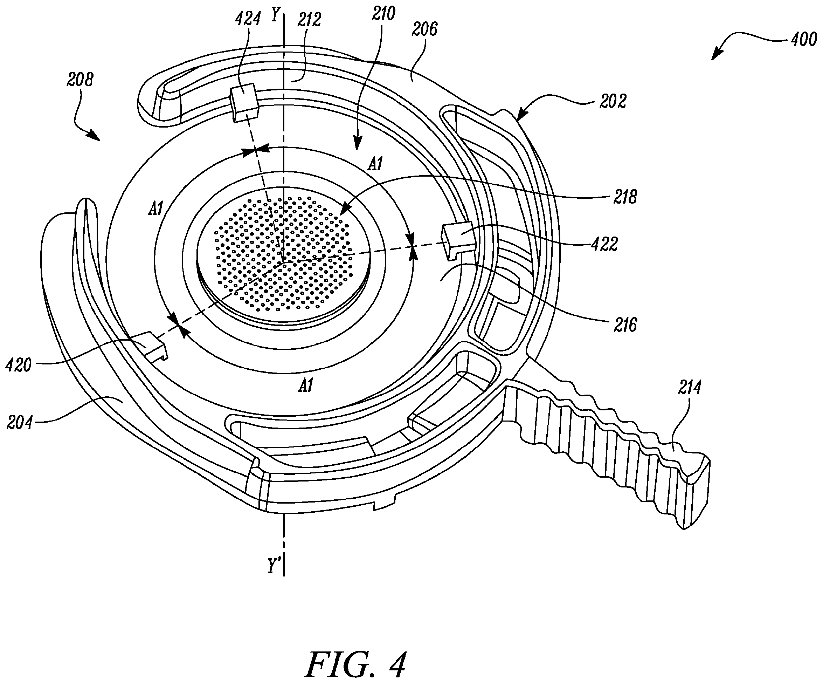

Referring to FIG. 4, another embodiment of a microarray carrier 400 is illustrated. The microarray carrier 400 is substantially similar to the microarray carrier 200 as described with reference to FIG. 2. As such, the microarray carrier 400 includes the body 202, the cavity 210, the inner surface 212, the handle 214, and the microarray patch 216. The microarray carrier 400 also includes a plurality of tabs 420, 422, 424 disposed on the inner surface 212 and extending into the cavity 210. The plurality of tabs 420, 422, 424 is adapted to releasably receive the microarray patch 216 within the cavity 210. In the illustrated embodiment, the plurality of tabs 420, 422, 424 includes three tabs 420, 422, 424. Each of the tabs 420, 422, 424 is disposed on the inner surface 212 of the body 202. Further, the tabs 420, 422, 424 are disposed equiangularly around the cavity 210. As such, the angle "A1" between the adjoining tabs 420, 422, 424 measures approximately 120°. In other embodiments, an actual value of the angle "A1" between the adjoining tabs 420, 422, 424 may vary, based on application requirements. Further, each of the tabs 420, 422, 424 has a configuration substantially similar to the configuration of the tab 220, 311, 321, 331, 341, 351, 361, 371 of the microarray carrier 200. As such each of the tabs 420, 422, 424 may have any configuration as described with reference to FIGS. 3A to 3I.

The microarray carrier 400 provides a substantially smaller contact area between the microarray carrier 400 and the microarray patch 216. As such, the microarray carrier 400 may be manufactured using engineered plastics, such as different types of polypropylene, without providing excessive adhesion between the microarray carrier 400 and the microarray patch 216 and, thus, improved detachability and uniform application of the microarray patch 216 on the skin surface of the subject. Also, the different types of polypropylene may provide increased dimensional stability during manufacturing of the microarray carrier 400.

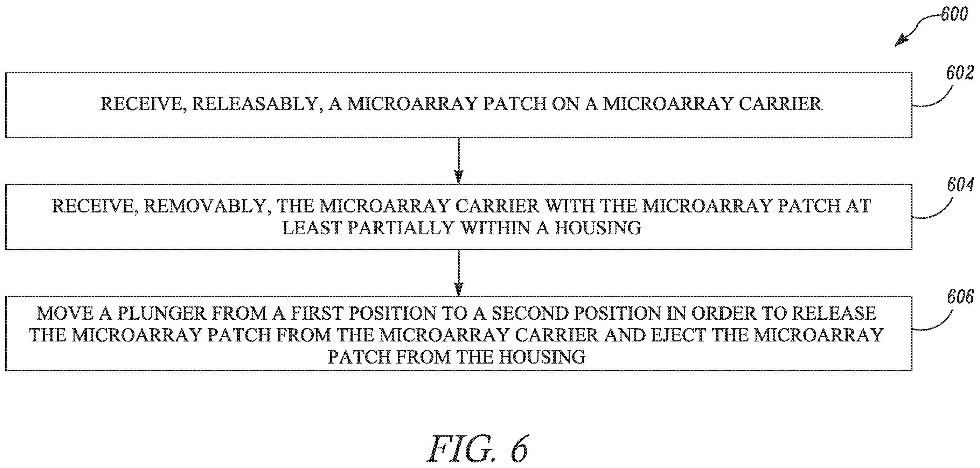

Referring to FIGS. 5A and 5B, cross-sectional views of different assembled positions of the microarray applicator 100 are illustrated. More specifically, in FIG. 5A, the microarray applicator 100 is shown with the plunger 110 in a first position "P1", and in FIG. 5B, the microarray applicator 100 is shown with the plunger 110 in a second position "P2". Referring to FIG. 6, a flowchart of a method 600 of using the microarray applicator 100 is illustrated. The method 600 will now be described with combined reference to FIGS. 5A, 5B, and 6. In the assembled position of the microarray applicator 100, the plunger system 114 is disposed on the housing 102, such that the plunger 110 is adapted to be movably received within the housing 102. More specifically, the plunger 110 is received within the bore 108 of the housing 102, such that the axis X-X' coincides with the axis Z-Z'.

At step 602, the microarray patch 216 is releasably received on the microarray carrier 200. More specifically, the microarray patch 216 is releasably supported on the support surface 228, 230, 232, 234 of each of the tabs 220, 222, 224, 226. It should be noted that, in other embodiments, the microarray carrier 200 may alternatively be the microarray carrier 400 as described with reference to FIG. 4. The area "SA" of the support surface 228, 230, 232, 234 of each of the tabs 220, 222, 224, 226 provides a substantially reduced support area relative to the projected area "PA" of each of the tabs 220, 222, 224, 226 in order to support the microarray patch 216 within the cavity 210. As such, each of the support surfaces 228, 230, 232, 234 reduces contact between each of the tabs 220, 222, 224, 226 and the microarray patch 216 and, thus, reduces adhesion between each of the tabs 220, 222, 224, 226 and the microarray patch 216 during detachment.

At step 604, the microarray carrier 200 is removably received at least partially within the housing 102. More specifically, the microarray carrier 200 is removably received within the housing 102, such that the body 202 of the microarray carrier 200 is disposed within the housing 102 and in association with the plunger 110, and the handle 214 is disposed outside the housing 102. Also, the microarray carrier 200 is removably received within the receiving portion 112 of the housing 102, such that the axis Y-Y' coincides with the axis X-X' and the axis Z-Z'. Accordingly, in the assembled position, the microarray applicator 100 includes the microarray carrier 200 with the microarray patch 216 disposed within the housing 102 and the plunger 110 disposed above the microarray patch 216 within the housing 102. Further, the microarray carrier 200 is aligned on the skin surface of the subject. More specifically, the microarray applicator 100 is aligned on a location on the skin surface of the subject where deposition of the microarray patch 216 may be required.

At step 606, the plunger 110 is moved from the first position "P1" to the second position "P2" in order to release the microarray patch 216 from the microarray carrier 200. More specifically, the plunger 110 is moved along the axis Z-Z' in a direction "D1" in order to move the plunger 110 from the first position "P1" to the second position "P2". As the plunger 110 moves from the first position "P1" toward the second position "P2", the plunger 110 contacts the microarray patch 216. As the plunger 110 further moves to the second position "P2", the microarray patch 216 is detached from each of the tabs 220, 222, 224, 226 of the microarray carrier 200 and moved in the direction "D1" along with the plunger 110 toward the skin surface. As the plunger 110 reaches the second position "P2" along with the microarray patch 216, the microarray patch 216 is ejected from the cavity 210 of the microarray carrier 200 and the housing 102 and is deposited on the skin surface of the subject. In some situations, contact the microneedles 218 and the skin surface can be maintained by continuing to apply pressure on the plunger 110 in the direction "D1". The microarray applicator 100 with the microarray carrier 200 present within the housing 102 may now be removed from the skin surface of the subject with the microarray patch 216 deposited on the skin surface.

Additionally, the microarray applicator 100 may be reset for reuse after deposition of the microarray patch 216 on the skin surface. For reuse, the microarray carrier 200 without the microarray patch 216 therein may be removed from the housing 102 using the handle 214. Also, the plunger 110 may be moved from the second position "P2" to the first position "P1" in a direction "D2" by action of one or more springs 118, 120, 122. Further, the microarray carrier 200 with a new patch (not shown), similar to the microarray patch 216, therein may be inserted in the housing 102 for reuse of the microarray applicator 100 for depositing the new patch on the skin surface of the subject. In some embodiments, a new carrier (not shown), similar to the microarray carrier 200 or the microarray carrier 400, with the new patch therein may be inserted in the housing 102 for reuse of the applicator 100 for depositing the new patch on the skin surface of the subject.

It should be noted that the microarray carriers described herein may be manufactured using different types of engineered plastics, such as different types of polypropylene, other alternate materials, and so on, in turn, improving compatibility and manufacturing flexibility. It should also be noted that while microarray carriers are described herein for use with a particular applicator, they are also envisioned for use with other applicators.

## Claims

1. A microarray carrier (200, 400) for use with a microarray applicator (100), the microarray carrier comprising:
a body (202) defining a cavity (210); and
a plurality of tabs (220, 311, 321, 331, 341, 351, 361, 371, 420) disposed on the body (202) and extending into the cavity (210), each of the plurality of tabs (220, 311, 321, 331, 341, 351, 361, 371, 420) having a contour line (L-L') extending along a length thereof from a proximal end of the tab adjacent the body to a support surface (228, 316, 326, 336, 346, 356, 366, 376) spaced apart from the body (202), the support surface (228, 316, 326, 336, 346, 356, 366, 376) inclined with respect to the contour line (L-L') where the contour line intersects the support surface (228, 316, 326, 336, 346, 356, 366, 376), wherein a projected area of each of the plurality of tabs (220, 311, 321, 331, 341, 351, 361, 371, 420) onto a horizontal plane (HP) ofthe cavity (210) is greater than an area of the support surface (228, 316, 326, 336, 346, 356, 366, 376) and wherein the support surface (228, 316, 326, 336, 346, 356, 366, 376) is aligned non-perpendicular to the horizontal plane (HP).

2. The microarray carrier (200, 400) of claim 1, wherein each of the plurality of tabs (220, 311, 321, 331, 341, 351, 361, 371, 420) further includes a first portion (302, 312, 322, 332, 342, 352) extending from the body and a second portion (304, 314, 324, 334, 344, 354, 374) inclined to the first portion, the second portion (304, 314, 324, 334, 344, 354, 374) defining the support surface (228, 316, 326, 336, 346, 356, 366, 376).

3. The microarray carrier (200, 400) of claim 2, wherein an angle between the first portion (302, 312, 322, 332, 342, 352) and the second portion (304, 314, 324, 334, 344, 354, 374) is from about 80 degrees to about 100 degrees.

4. The microarray carrier (200, 400) of claim 1, wherein the contour line (L-L') of each tab (220, 311, 321, 331, 341, 351, 361, 371, 420) is an axis inclined obliquely relative to the horizontal plane (HP) of the cavity (210).

5. The microarray carrier (200, 400) of any of the preceding claims, wherein each of the plurality of tabs (351, 361) is at least partially curved.

6. The microarray carrier (200, 400) of any of the preceding claims, wherein each of the plurality of tabs (220, 311, 321, 331, 341, 351, 361, 371, 420) has a substantially rectangular cross-section.

7. The microarray carrier (200, 400) of any of the preceding claims, wherein the contour line (L-L') at the distal end of the tab (220, 331, 341, 351,361, 420) is substantially normal to the support surface (228, 336, 346, 356, 366, 376).

8. The microarray carrier (200, 400) of any of the preceding claims, wherein the support surface (228, 316, 326, 336, 346, 356, 366, 376) is disposed at an end of each of the plurality of tabs.

9. The microarray carrier (200, 400) of any of the preceding claims, wherein the plurality of tabs (420) includes three tabs (420, 422, 424), the three tabs being equiangularly disposed around the cavity.

10. The microarray carrier (200, 400) of any of the preceding claims, wherein the cavity (210) has a substantially annular shape.

11. The microarray carrier (200, 400) of any of the preceding claims further includes a handle (214) extending from the body (202).

12. The microarray carrier (200, 400) of any of the preceding claims wherein the support surface (228, 316, 326, 336, 346, 356, 366, 376) is aligned essentially parallel to the horizontal plane (HP).

13. A microarray applicator (100) comprising the microarray carrier (200, 400) of any of the preceding claims and:
a housing (102); and
a plunger (110) adapted to be movably disposed within the housing (102),
wherein the microarray carrier (200, 400) is removably and at least partially disposed within the housing.

14. The microarray applicator (100) of claim 13 further comprising a microarray patch (216) releasably retained by the microarray carrier (200, 400), wherein the microarray patch (216) is releasably supported by the support surface (228, 316, 326, 336, 346, 356, 366, 376) of each of the plurality of tabs (220, 311, 321, 331, 341, 351, 361, 371, 420).

## Patentansprüche

1. Mikroarray-Träger (200, 400) zur Verwendung mit einem Mikroarray-Applikator (100), wobei der Mikroarray-Träger aufweist:
einen Körper (202), der einen Hohlraum (210) definiert; und
mehrere Ansätze (220, 311, 321, 331, 341, 351, 361, 371, 420), die auf dem Körper (202) angeordnet sind und sich in den Hohlraum (210) erstrecken, wobei jeder der mehreren Ansätze (220, 311, 321, 331, 341, 351, 361, 371, 420) eine Konturlinie (L-L') hat, die sich über eine Länge davon von einem proximalen Ende des Ansatzes benachbart zum Körper zu einer Stützfläche (228, 316, 326, 336, 346, 356, 366, 376) erstreckt, die vom Körper (202) beabstandet ist, wobei die Stützfläche (228, 316, 326, 336, 346, 356, 366, 376) im Hinblick auf die Konturlinie (L-L') dort geneigt ist, wo die Konturlinie die Stützfläche (228, 316, 326, 336, 346, 356, 366, 376) schneidet, wobei ein Projektionsbereich jedes der mehreren Ansätze (220, 311, 321, 331, 341, 351, 361, 371, 420) auf eine Horizontalebene (HP) des Hohlraums (210) größer ist als ein Bereich der Stützfläche (228, 316, 326, 336, 346, 356, 366, 376) und wobei die Stützfläche (228, 316, 326, 336, 346, 356, 366, 376) nicht senkrecht zur Horizontalebene (HP) ausgerichtet ist.

2. Mikroarray-Träger (200, 400) nach Anspruch 1, wobei jeder der mehreren Ansätze (220, 311, 321, 331, 341, 351, 361, 371, 420) ferner aufweist: einen ersten Abschnitt (302, 312, 322, 332, 342, 352), der sich vom Körper erstreckt, und einen zweiten Abschnitt (304, 314, 324, 334, 344, 354, 374), der zum ersten Abschnitt geneigt ist, wobei der zweite Abschnitt (304, 314, 324, 334, 344, 354, 374) die Stützfläche (228, 316, 326, 336, 346, 356, 366, 376) definiert.

3. Mikroarray-Träger (200, 400) nach Anspruch 2, wobei ein Winkel zwischen dem ersten Abschnitt (302, 312, 322, 332, 342, 352) und dem zweiten Abschnitt (304, 314, 324, 334, 344, 354, 374) etwa 80 Grad bis etwa 100 Grad beträgt.

4. Mikroarray-Träger (200, 400) nach Anspruch 1, wobei die Konturlinie (L-L') jedes Ansatzes (220, 311, 321, 331, 341, 351, 361, 371, 420) eine Achse ist, die relativ zur Horizontalebene (HP) des Hohlraums (210) schräg geneigt ist.

5. Mikroarray-Träger (200, 400) nach einem der vorstehenden Ansprüche, wobei jeder der mehreren Ansätze (351, 361) zumindest teilweise gekrümmt ist.

6. Mikroarray-Träger (200, 400) nach einem der vorstehenden Ansprüche, wobei jeder der mehreren Ansätze (220, 311, 321, 331, 341, 351, 361, 371, 420) einen im Wesentlichen rechtwinkligen Querschnitt hat.

7. Mikroarray-Träger (200, 400) nach einem der vorstehenden Ansprüche, wobei die Konturlinie (L-L') am distalen Ende des Ansatzes (220, 331, 341, 351, 361, 420) im Wesentlichen senkrecht zur Stützfläche (228, 336, 346, 356, 366, 376) ist.

8. Mikroarray-Träger (200, 400) nach einem der vorstehenden Ansprüche, wobei die Stützfläche (228, 316, 326, 336, 346, 356, 366, 376) an einem Ende jedes der mehreren Ansätze angeordnet ist.

9. Mikroarray-Träger (200, 400) nach einem der vorstehenden Ansprüche, wobei die mehreren Ansätze (420) drei Ansätze (420, 422, 424) aufweisen, wobei die drei Ansätze um den Hohlraum gleichwinklig angeordnet sind.

10. Mikroarray-Träger (200, 400) nach einem der vorstehenden Ansprüche, wobei der Hohlraum (210) im Wesentlichen eine Ringform hat.

11. Mikroarray-Träger (200, 400) nach einem der vorstehenden Ansprüche, der ferner einen Griff (214) aufweist, der sich vom Körper (202) erstreckt.

12. Mikroarray-Träger (200, 400) nach einem der vorstehenden Ansprüche, wobei die Stützfläche (228, 316, 326, 336, 346, 356, 366, 376) im Wesentlichen parallel zur Horizontalebene (HP) ausgerichtet ist.

13. Mikroarray-Applikator (100) mit dem Mikroarray-Träger (200, 400) nach einem der vorstehenden Ansprüche und:
einem Gehäuse (102); und
einem Kolben (110), der geeignet ist, im Gehäuse (102) beweglich angeordnet zu sein,
wobei der Mikroarray-Träger (200, 400) entfernbar und zumindest teilweise im Gehäuse angeordnet ist.

14. Mikroarray-Applikator (100) nach Anspruch 13, ferner mit einem Mikroarray-Patch (216), das durch den Mikroarray-Träger (200, 400) lösbar festgehalten wird, wobei das Mikroarray-Patch (216) durch die Stützfläche (228, 316, 326, 336, 346, 356, 366, 376) jedes der mehreren Ansätze (220, 311, 321, 331, 341, 351, 361, 371, 420) lösbar gestützt wird.

## Revendications

1. Support de microréseau (200, 400) destiné à être utilisé avec un applicateur de microréseau (100), le support de microréseau comprenant :
un corps (202) définissant une cavité (210) ; et
une pluralité de languettes (220, 311, 321, 331, 341, 351, 361, 371, 420) disposées sur le corps (202) et s'étendant dans la cavité (210), chacune de la pluralité de languettes (220, 311, 321, 331, 341, 351, 361, 371, 420) ayant une ligne de contour (L-L') s'étendant le long de sa longueur à partir d'une extrémité proximale de la languette adjacente au corps jusqu'à une surface de support (228, 316, 326, 336, 346, 356, 366, 376) espacée du corps (202), la surface de support (228, 316, 326, 336, 346, 356, 366, 376) inclinée par rapport à la ligne de contour (L-L') où la ligne de contour coupe la surface de support (228, 316, 326, 336, 346, 356, 366, 376), dans lequel une zone projetée de chacune de la pluralité de languettes (220, 311, 321, 331, 341, 351, 361, 371, 420) sur un plan horizontal (HP) de la cavité (210) est supérieure à une zone de la surface de support (228, 316, 326, 336, 346, 356, 366, 376) et dans lequel la surface de support (228, 316, 326, 336, 346, 356, 366, 376) est alignée de manière non perpendiculaire par rapport au plan horizontal (HP).

2. Support de microréseau (200, 400) selon la revendication 1, dans lequel chacune de la pluralité de languettes (220, 311, 321, 331, 341, 351, 361, 371, 420) comprend en outre une première partie (302, 312, 322, 332, 342, 352) s'étendant à partir du corps et une deuxième partie (304, 314, 324, 334, 344, 354, 374) inclinée par rapport à la première partie, la deuxième partie (304, 314, 324, 334, 344, 354, 374) définissant la surface de support (228, 316, 326, 336, 346, 356, 366, 376).

3. Support de microréseau (200, 400) selon la revendication 2, dans lequel un angle entre la première partie (302, 312, 322, 332, 342, 352) et la deuxième partie (304, 314, 324, 334, 344, 354, 374) est d'environ 80 degrés à environ 100 degrés.

4. Support de microréseau (200, 400) selon la revendication 1, dans lequel la ligne de contour (L-L') de chaque languette (220, 311, 321, 331, 341, 351, 361, 371, 420) est un axe incliné de manière oblique par rapport au plan horizontal (HP) de la cavité (210).

5. Support de microréseau (200, 400) selon l'une quelconque des revendications précédentes, dans lequel chacune de la pluralité de languettes (351, 361) est au moins partiellement courbe.

6. Support de microréseau (200, 400) selon l'une quelconque des revendications précédentes, dans lequel chacune de la pluralité de languettes (220, 311, 321, 331, 341, 351, 361, 371, 420) a une section transversale sensiblement rectangulaire.

7. Support de microréseau (200, 400) selon l'une quelconque des revendications précédentes, dans lequel la ligne de contour (L-L'), au niveau de l'extrémité distale de la languette (220, 331, 341, 351, 361, 420) est sensiblement normale par rapport à la surface de support (228, 336, 346, 356, 366, 376).

8. Support de microréseau (200, 400) selon l'une quelconque des revendications précédentes, dans lequel la surface de support (228, 316, 326, 336, 346, 356, 366, 376) est disposée à une extrémité de chacune de la pluralité de languettes.

9. Support de microréseau (200, 400) selon l'une quelconque des revendications précédentes, dans lequel la pluralité de languettes (420) comprend trois languettes (420, 422, 424), les trois languettes étant disposées, de manière équiangulaire, autour de la cavité.

10. Support de microréseau (200, 400) selon l'une quelconque des revendications précédentes, dans lequel la cavité (210) a une forme sensiblement annulaire.

11. Support de microréseau (200, 400) selon l'une quelconque des revendications précédentes, comprenant en outre une poignée (214) s'étendant à partir du corps (202).

12. Support de microréseau (200, 400) selon l'une quelconque des revendications précédentes, dans lequel la surface de support (228, 316, 326, 336, 346, 356, 366, 376) est alignée de manière essentiellement parallèle au plan horizontal (HP).

13. Applicateur de microréseau (100) comprenant le support de microréseau (200, 400) selon l'une quelconque des revendications précédentes, et :
un boîtier (102) ; et
un piston plongeur (110) adapté pour être disposé, de manière mobile, à l'intérieur du boîtier (102),
dans lequel le support de microréseau (200, 400) est disposé, de manière amovible et au moins partiellement à l'intérieur du boîtier.

14. Applicateur de microréseau (100) selon la revendication 13, comprenant en outre un patch de microréseau (216) retenu, de manière libérable, par le support de microréseau (200, 400), dans lequel le patch de microréseau (216) est supporté, de manière libérable, par la surface de support (228, 316, 326, 336, 346, 356, 366, 376) de chacune de la pluralité de languettes (220, 311, 321, 331, 341, 351, 361, 371, 420).
